Europäisches Patentamt

European Patent Office

Office européen des brevets

(19)

(11) Veröffentlichungsnummer: **0 223 033 B1**

## (12) EUROPÄISCHE PATENTSCHRIFT

(45) Veröffentlichungstag der Patentschrift: **10.07.91**

(51) Int. Cl.5: **A61K 35/14**, A61K 9/16

(21) Anmeldenummer: **86113714.9**

(22) Anmeldetag: **03.10.86**

(54) Verfahren zum gleichzeitigen Trocknen und Granulieren von Extraktstoffen aus enteiweisstem Kälberblut, nach diesem Verfahren erhaltenes Granulat und dessen Verwendung zur Herstellung von pharmazeutischen Präparaten.

(30) Priorität: **04.10.85 DE 3535536**

(43) Veröffentlichungstag der Anmeldung:
**27.05.87 Patentblatt 87/22**

(45) Bekanntmachung des Hinweises auf die Patenterteilung:
**10.07.91 Patentblatt 91/28**

(84) Benannte Vertragsstaaten:
**AT BE CH DE ES FR GB GR IT LI LU NL SE**

(56) Entgegenhaltungen:
**EP-A- 0 106 309**

**CHEMICAL ENGINEERING PROGRESS, Band 79, Nr. 4, April 1983, American Institute of Chemical Engineers, New York, NY (US); S.MORTENSEN et al., Seiten 37-42**

**CHEMICAL ABSTRACTS, Band 88, Nr. 26, 26 Juni 1978, Columbus, OH (US); T.SCHOEFER et al., Seite 401, Nr. 197584q**

**CHEMICAL ABSTRACTS, Band 84, Nr. 15, 12 April 1976, Columbus, OH (US); Seite 455, Nr. 104083e**

**H. SUCKER et al.: "Pharmazeutische Technologie", Ausgabe 1, 1978, Seiten 320-333,404-413, Georg Thieme Verlag, Stuttgart (DE)**

(73) Patentinhaber: **Hormon-Chemie München GmbH**
**Freisinger Landstrasse 74**
**W-8000 München 45(DE)**

(72) Erfinder: **Klug, Otto**
**Frauenstr. 27**
**W-8059 Langenpreising(DE)**
Erfinder: **Schlünken, Heinrich, Dr.**
**Boschweg 1c**
**A-4020 Linz(AT)**
Erfinder: **Siegel, Dietmar, Dr.**
**Gartenstr. 81**
**W-8050 Freising(DE)**

(74) Vertreter: **Kunz, Ekkehard, Dr.**
**Chemie Holding AG Patentwesen St. Peter-Strasse 25**
**A-4021 Linz(AT)**

## Beschreibung

Die Erfindung betrifft ein Wirbelschichtverfahren zur gleichzeitigen Gewinnung der Trockensubstanz aus wässerigen Lösungen von Extraktstoffen aus enteiweißtem Kälberblut und Herstellung eines zu festen Arzneiformen verpreßbaren Granulats, welches mindestens 50 Gew. % Extraktstoffe bezogen auf das Gesamttrockengewicht des Granulats enthält, und die Verwendung eines nach diesem Verfahren erhaltenen Granulats zur Herstellung von pharmazeutischen Präparaten, die Extraktstoffe aus enteiweißtem Kälberblut als Wirksubstanz enthalten.

Es ist bekannt, daß Extraktstoffe aus enteiweißtem Blut junger Kälber, welche die niedermolekularen Bestandteile des Kälberbluts enthalten, bei der Behandlung schlecht heilender Wunden eine Verbesserung der Durchblutung des Gewebes bewirken und damit eine Abheilung der Wunde beschleunigen. Weiters finden die Extraktstoffe auch bei cerebralen Durchblutungs- und Stoffwechselstörungen Anwendung, vg. Schnellen, Med. Welt, Bd. 19, S 198 (1968). Pharmazeutische Präparate, die solche Extraktstoffe enthalten, sind unter dem Namen "Actovegin" der Fa. Hormonchemie, München, oder unter der Bezeichnung "Actihaemyl" der Fa. Solko, Basel, im Handel.

Die Extraktstoffe an enteiweißtem Kälberblut sind als Trockensubstanz äußerst wärmeempfindlich. Bei längerer thermischer Belastung über 30° C entsteht eine braune Verfärbung, die wahrscheinlich durch eine Reaktion von Glucose mit Aminosäuren und Peptiden hervorgerufen wird (Maillard Reaktion), wobei sich ein Restwassergehalt in der Trockensubstanz von mehr als 4 Gew. % besonders nachteilig auswirkt und zur Bildung einer braunen, zähen Masse führt.

Bei der Isolierung aus dem Kälberblut fallen die Extraktstoffe in wässerigen Lösungen unterschiedlicher Konzentration an. In der DE-PS-1,076.888 ist nur die Herstellung von injizierbaren Präparaten mit einer Konzentration von 30 bis 60 mg Trockensubstanz/ml Lösung durch Einengung von Lösungen der Extraktstoffe beschrieben. Für die Gewinnung der Trockensubstanz selbst zur Herstellung von festen Arzneiformen kommen wegen der großen Thermolabilität der Extraktstoffe nur schonende Trocknungsmethoden in Betracht. Aus der AT-PS-330.953 ist ein Verfahren zur Herstellung von Trockenpräparaten von Kälberblutextrakten bekannt, bei dem die Extraktstoffe mit einem Adsorbens, z.B. einem hochdispersem Siliciumdioxid, vermischt werden, worauf das entstehende thixotrope Gel im Vakuum getrocknet wird. Dieses Verfahren hat den Nachteil, daß der wässerigen Lösung der Extraktstoffe vor der Trocknung extraktfremde Begleitstoffe zugesetzt werden müssen, da ohne solche Zusatzstoffe die Herstellung einer einwandfreien Trockensubstanz auf diesem Weg undurchführbar ist.

Eine verbesserte Alternative zur Adsorption der Blutextraktwirkstoffe an feste Stoffe besteht gemäß der AT-PS-330.953 darin, daß man die Trockensubstanz durch Gefriertrocknung (Lyophilisation) von etwa 5 bis 10%igen wässerigen Lösungen der Extraktstoffe gewinnt. Neben dem Umstand, daß die Gefriertrocknung von größeren Volumina der wässerigen Lösungen sehr zeitraubend und energieaufwendig ist, hat die Gefriertrocknung den Nachteil, daß die anfallenden Lyophilisate ein sehr feines und stark hygroskopisches Pulver darstellen, welches für die Herstellung von festen Arzneiformen durch Direkttablettierung oder durch Trockengranulation und anschließendes Verpressen nicht geeignet ist.

Aus dem Lyophilisat wird ein für die Tablettierung geeignetes Granulat bisher durch nachträgliche Feuchtgranulierung mit Polyvinylpyrrolidon als Bindemittel und Carboxymethylstärke als Füllstoff hergestellt, wobei aber als Granulierflüssigkeit Wasser nicht verwendet werden kann, sondern auf organische Lösungsmittel, beispielsweise Isopropanol, zurückgegriffen werden muß. Organische Lösungsmittel werden aber wegen ihrer Toxizität für die Herstellung von oral applizierbaren Arzneiformen nur ungern verwendet. Außerdem nimmt das hygroskopische Lyophilisat während des Granuliervorganges Feuchtigkeit aus der Umgebungsluft auf, sodaß entweder unter klimatisierten Bedingungen bei niedriger relativer Luftfeuchtigkeit gearbeitet werden, oder das fertige Granulat einem mehrstündigen Trocknungsprozess bei etwa 40° C unterzogen werden muß, wobei die Extraktstoffe einer weiteren Temperaturbelastung ausgesetzt sind, die qualitätsmindernd wirkt und zur Verfärbung der fertigen Präparate während der Lagerung Anlaß gibt.

Der Erfindung liegt daher die technische Aufgabe zugrunde, sowohl das Verfahren zur Gewinnung der Trockensubstanz aus den bei der Isolierung der Extraktstoffe anfallenden wässerigen Lösungen als auch die Herstellung eines zu festen Arzneiformen verpreßbaren Granulats unter Vermeidung der oben angeführten Nachteile zu verbessern, wobei neben wirtschaftlichen Vorteilen eine gleichmäßige, verbesserte Produktqualität eine entscheidende Forderung ist. Insbesondere soll die Trockensubstanz während des gesamten Herstellungsvorganges unter schonenden Bedingungen gewonnen und ein preßfertiges Granulat mit einem hohen Gewichtsanteil an Extraktstoffen hergestellt werden, welches sich zu festen Arzneiformen mit hoher Stabilität verarbeiten läßt, die auch bei längerer Lagerung bei Raumtemperaturen keine Verfärbung zeigen und eine verbesserte Konstanz der Qualitätsparameter aufweisen.

Die Aufgabe wird gemäß der Erfindung gelöst mit einem Verfahren zur gleichzeitigen Gewinnung der Trockensubstanz aus wässerigen Lösungen von Extraktstoffen aus enteiweißtem Kälberblut und Herstellung eines zu festen Arzneiformen verpreßbaren Granulats, das mindestens 50 Gew. % Extraktstoffe bezogen auf das Gesamttrockengewicht des Granulats, ein Bindemittel und pharmazeutisch verträgliche Füll- und Trägerstoffe enthält, wobei das Verfahren dadurch gekennzeichnet ist, daß man in einem kontinuierlichen Prozeß in einem Wirbelschichtgranulator auf eine vorgegebene Menge der Füll-und Trägerstoffe in fluidisiertem Zustand ein die gewünschte Extraktstoffmenge enthaltendes Volumen einer konzentrierten wässerigen Lösung, die auf 10 Gew. Teile der Extraktstoffe zusätzlich 0.2 bis 2 Gew. Teile des Bindemittels in gelöstem Zustand enthält, aufsprüht, wobei man während des Aufsprühens des gesamten Lösungsvolumens oder zumindest des größeren Teiles davon die Temperatur des einströmenden Fluidisiergases und die Sprühgeschwindigkeit so einstellt, daß bei einer Wirbelschichttemperatur von 25 bis 30° C und einem Wassergehalt des Wirbelschichtgutes von 15 bis 20 Gew. % die eingesprühte und verdampfende Wassermenge einander etwa entsprechen, keine Formgebung stattfindet und das Wirbelschichtgut pulverförmig bleibt, zum Agglomerieren und Granulieren des so erhaltenen Mischguts noch während oder nach Beendigung des Aufsprühens der Extraktstoffe den Wassergehalt in der Wirbelschicht auf 35 bis 45 Gew. % erhöht und anschließend das gebildete Granulat nach Erreichen der gewünschten Granulatgröße durch Erhöhen der Temperatur des einströmenden Fluidisiergases bei einer Wirbelschichttemperatur von 30 bis höchstens 45° C der Kurzzeittrocknung unterwirft.

Die Vorteile des erfindungsgemäßen Verfahrens liegen einerseits in der Erhöhung der Produktqualität des erhaltenen Granultas, welches sich nach dem Austrag aus dem Wirbelschichtgranulator entweder direkt oder nach Zusatz von weiteren pharmazeutisch verträglichen Hilfsstoffen zu gut lagerungsfähigen festen Arzneiformen mit hoher Stabilität, großer Gewichtskonstanz und ausgezeichneten galenischen Eigenschaften verpressen läßt. Andererseits ermöglicht das erfindungsgemäße Verfahren sowohl die schonende Gewinnung der Trockensubstanz aus großen Volumina wässeriger Lösungen von Extraktstoffen aus enteiweißtem Kälberblut als auch die Herstellung eines preßfertigen Granulats in einem einzigen kontinuierlichen Prozeß auf eine wesentlich einfachere und wirtschaftlichere Weise als es mit dem bekannten Verfahren bisher der Fall war.

Für die Ausführung des erfindungsgemäßen Verfahrens eignen sich handelsübliche Wirbelschichtgranulatoren. Solche Wirbelschichtgranulatoren bestehen im wesentlichen aus einem Materialbehälter, in den das Fluidisiergas seitlich oder von unten einströmt, wobei sowohl die Strömungsgeschwindigkeit als auch die Temperatur des einströmenden Fluidisiergases regelbar ist. Weiters verfügen diese Geräte über einen oder mehrere Sprühköpfe in Form von Einstoff- oder Zweistoffdüsen, über die reine Flüssigkeiten und Lösungen mittels einer Pumpe mit einstellbarer Sprühgeschwindigkeit in den Materialbehälter eingesprüht werden können und die so angeordnet sind, daß die zerstäubte Flüssigkeit oder Lösung direkt in die Wirbelschicht hineinströmt. Ein mechanisches Rühr- oder Zerhackerwerk auf dem Behälterboden oder ein Rüttelwerk soll bei Bedarf für einen zusätzlichen Mischeffekt sorgen.

Zur praktischen Durchführung des Verfahrens wird eine vorgegebene Menge der Füll- und Trägerstoffe, die sich nach dem gewünschten Gewichtsanteil dieser Zusatzstoffe im fertigen Granulat richtet und beim erfindungsgemäßen Verfahren in jedem Fall weniger als 50 Gew. %, bevorzugt 25 bis 45 Gew. % bezogen auf das Gesamttrockengewicht des fertigen Granulats beträgt, im Materialbehälter vorgelegt und durch Einströmen des Fluidisiergases eine gleichmäßige Wirbelschicht gebildet. Für die Herstellung des erfindungsgemäßen Granulats eignen sich vor allem in der Galenik übliche Füll- und Trägerstoffe mit kleiner Teilchengröße, die gleichzeitig eine große Oberfläche besitzen und in Wasser unlöslich oder nur schwer löslich sind. Als geeignete Füll- und Trägerstoffe mit den oben beschriebenen Eigenschaften sind beispielsweise mikrokristalline Cellulose (im Handel z.B. unter dem Namen Avicel PH 101 erhältlich), quervernetzte Natrium-carboxymethylcellulose (im Handel z.B. unter dem Namen Ac-DI-Sol erhältlich), quervernetztes Polyvinylpyrrolidon (im Handel z.B. unter dem Namen Polyplasdone erhältlich) oder Gemische dieser Stoffe zu nennen, wobei die Mischungsverhältnisse in einem weiten Bereich beliebig gewählt werden können und hier wiederum Mischungen aus gleichen Gewichtsteilen dieser Stoffe bevorzugt sind. Ganz besonders bewährt haben sich für diesen Zweck mikrokristalline Cellulose oder quervernetzte Natriumcarboxymethylcellulose.

Als Fluidisiergas kann man in einfachster Weise ohne Nachteil für die Qualität und Beständigkeit der Extraktstoffe trockene Luft verwenden. Zur Bildung der Wirbelschicht eignen sich aber selbstverständlich auch andere Gase, die gegenüber den Extraktstoffen inert sind, wie beispielsweise Stickstoff. Der Gesamtdurchsatz an Fluidisiergas pro Stunde hängt von der Größe des Wirbelschichtgranulators ab und beträgt bei einem Nennvolumen des Materialbehälters von 60 Liter beispielsweise 500 - 3000 $m^3$/h. Sobald sich infolge des Strömungsdruckes eine gleichmäßige Wirbelschicht gebildet hat, kann mit dem Aufsprühen der wässerigen Lösung der Extrakstoffe begonnen werden. Man verwendet hiezu vorteilhafterweise konzentrierte wässerige Lösungen, die bis zu 50 Gew. %, vorzugsweise 15 bis 25 Gew. %, an Extraktstoffen enthalten und deren Volumen so berechnet ist, daß die Gesamtmenge der aufgesprühten Extraktstoffe mindestens 50 Gew. %, vorzugsweise 53 - 70 Gew. % bezogen auf das Gesamttrockengewicht des fertigen Granulats beträgt.

Diese Lösungen enthalten auf 10 Gew. Teile der Extraktstoffe zusätzlich 0.2 bis 2 Gew. Teile eines wasserlöslichen Bindemittels, vorzugsweise 0.5 bis 1 Gewichtsteile. Der Gewichtsanteil des Bindemittels im fertigen Granulat beträgt dann beispielsweise 1 bis 10 Gew. %, vorzugsweise 2 bis 6 Gew. %, bezogen auf das Gesamttrockengewicht des Granulats.

Als Bindemittel eignen sich für die Herstellung des erfindungsgemäßen Granulats in der Galenik übliche wasserlösliche Bindemittel, beispielsweise vorgelatinierte Stärke, wasserlösliche Cellulose, wie Methylcellulose, Hydroxypropylcellulose, Hydroxypropylmethylcellulose oder andere wasserlösliche hochmolekulare Verbindungen wie Polyvinylalkohol, Polyvinylpyrrolidon und dergleichen. Besonders bevorzugt werden als Bindemittel Polymere des 1-Vinyl-2-pyrrolidons, die beispielsweise unter dem Namen Kollidon K 25 oder Kollidon 90 im Handel sind, verwendet.

Die Temperatur des einströmenden Fluidisiergases während des Sprühvorganges und die Sprühgeschwindigkeit, mit der die wässerige Lösung der Extraktstoffe gemeinsam mit dem Bindemittel auf die fluidisierten Füll- und Trägerstoffe aufgesprüht wird, hängt von der vorgelegten Menge der Hilfsstoffe und dem Nennvolumen des Materialbehälters des Wirbelschichtgranulators ab. Die Temperatur und die Sprühgeschwindigkeit werden in dieser Phase des Verfahrens so aufeinander abgestimmt, daß sich bei einer Wirbelschichttemperatur von 25 bis 30° C im Wirbelschichtgut ein stationärer Wassergehalt von 15 bis 20 Gew. %, bezogen auf das Gesamtgewicht, einstellt und die eingesprühte und verdampfende Wassermenge einander etwa entsprechen.

Hiezu sind je nach Menge der vorgelegten Füll- und Trägerstoffe und der Größe des Materialbehälters Fluidisiergastemperaturen von etwa 30 bis 80° C erforderlich.

Unter den angegebenen Bedingungen findet noch keine Formgebung statt. Die Füll- und Trägerstoffe bleiben aufnahmefähig für die Extraktstoffe, die in der Wirbelschicht vorteilhafterweise keiner wesentlichen Temperaturbelastung ausgesetzt sind, während der größte Teil des Wassers entfernt wird, und man erhält ein feines rieselfähiges Mischgut, welches in dieser ersten Phase des Verfahrens pulverförmig bleibt und kaum zur Agglomeration neigt.

Die Agglomeration des pulverförmigen Materials zum Aufbau des Granulats der gewünschten Größe wird beim erfindungsgemäßen Verfahren durch Erhöhung des Wassergehalts in der Wirbelschicht auf 35 bis 45 Gew. %, vorzugsweise auf 38 bis 40 Gew. %, erreicht. Dazu kann der Wassergehalt entweder noch während des Sprühvorgangs, wenn der größere Teil der gewünschten Menge an Extraktstoffen bereits aufgesprüht ist, oder nach Beendigung des Aufsprühens der Extraktstoffe erhöht werden. In einer besonders vorteilhaften Ausführungsform des Verfahrens wird der größere Teil der Lösung, die die Extraktstoffe und das Bindemittel enthält, beispielsweise je nach der Konzentration an Extraktstoffen 75 bis 85 Volums % des gesamten Volumens der aufzusprühenden Lösung, unter den oben angegebenen Bedingungen, die nicht zur Agglomeration des Mischguts führen, aufgesprüht, während beim Aufsprühen des restlichen Teils des Lösungsvolumens die Temperatur des einströmenden Fluidisiergases gesenkt und/oder Sprühgeschwindigkeit vergrößert wird, sodaß eine größere Wassermenge eingesprüht wird als gleichzeitig verdampfen kann und in der Folge der Wassergehalt in der Wirbelschicht auf den zum Agglomerieren und Granulieren erforderlichen Wert ansteigt.

Es ist aber auch möglich das gesamte Lösungsvolumen unter Bedingungen, die nicht zur Agglomeration führen, aufzusprühen und dann den Wassergehalt in der Wirbelschicht durch Einsprühen von reinem Wasser auf den zum Granulieren erforderlichen Wert zu erhöhen.

Sobald der Wassergehalt in der Wirbelschicht den Wert von etwa 35 Gew. % überschreitet, setzt die Agglomeration ein und es wird ein gleichmäßiges, einheitliches Granulat gebildet.

Während des gesamten Granuliervorganges wird im Wirbelschichtgut ein Wassergehalt innerhalb der oben angegebenen Grenzen aufrecht erhalten. Je nach Einstellung des Wassergehaltes kann man nach dem erfindungsgemäßen Verfahren in reproduzierbarer Weise Granulate erhalten, die einen Durchmesser im Bereich von 0.03 bis 2 mm, bevorzugt von 0.05 bis 1 mm besitzen.

Wenn sich das Granulat der gewünschten Größe gebildet hat, wird durch Erhöhung der Temperatur des Fluidisiergases die Trocknungsphase eingeleitet. Man wählt die Temperatur des Fluidisiergases dabei zweckmäßigerweise so, daß zur schonenden Behandlung der Extraktstoffe die Granulattemperatur in der Wirbelschicht 30 bis 32° C nicht überschreitet. Die Trocknung ist beendet, sobald die Granulattemperatur ansteigt. Zur Entfernung des Restwasser kann man die Temperatur des Granulats für kurze Zeit, beispielsweise 5 bis 15 Minuten, ohne Beeinträchtigung der Stabilität der Extraktstoffe und der Qualität des Granulats auf 40 bis 45° C, vorteilhafterweise 40 bis 43° C, ansteigen lassen. Auf diese Weise erhält man Granulate mit einem Höchstwassergehalt von etwa 1.5 bis 3 Gew. % bezogen auf das Gesamtgewicht.

Das erfindungsgmäße Verfahren ist rationell, einfach und gut reproduzierbar. Zur Herstellung von 58 kg trockenem preßfertigem Granulat aus dem wässerigen Konzentrat der Extraktstoffe aus enteiweißtem Kälberblut benötigt man bei kontinuierlicher Arbeitsweise beispielsweise nur etwa 7 Stunden, während allein die nach dem Stand der Technik durchgeführte Gefriertrocknung der gleichen Menge etwa 7 bis 9 Tage beansprucht.

Das erfindungsgemäß hergestellte Granulat hat einen hohen Gewichtsanteil an Extraktstoffen und verfügt bei einer gleichmäßigen Korngröße über eine ausgezeichnete Fließfähigkeit, die seine Verarbeitung zu festen Arzneiformen mit großer Gewichtskonstanz und hoher Stabilität ermöglichen. Ein weiterer Gegenstand der Erfindung betrifft daher die Verwendung des erfindungsgemäßen Granulats zur Herstellung von festen Arzneiformen, die Extraktstoffe aus enteiweißtem Kälberblut als Wirksubstanz enthalten. Vorzugsweise werden Preßlinge, wie Tabletten, Dragees, Drageekerne oder andere Komprimate beliebiger Form und Größe hergestellt. Das erfindungsgemäß hergestellte Granulat kann dabei direkt oder nach Zumischung von weiteren in der Galenik bekannten Hilfsstoffen, wie Tablettenbindern, Füllstoffen, Konservierungsmitteln, Tablettensprengmitteln und dergleichen verpreßt werden. Die Art und Menge dieser Hilfsstoffe richtet sich nach der gewünschten mechanischen Festigkeit und Auflösungsgeschwindigkeit der Preßlinge.

Als solche Hilfsmittel zur Zumischung vor dem Verpressen eignen sich beispielsweise Stearate, wie Magnesiumstearat, Calciumstearat und dergleichen oder andere bei der Tablettierung üblicherweise verwendete Gleitmittel, beispielsweise Talkum oder Glycerinester gesättigter natürlicher Fettsäuren, wie diese beispielsweise unter dem Namen Precirol im Handel sind, in einer Menge von 1 bis 10 Gew. % bezogen auf das Gesamtgewicht des fertigen Präparats.

Die fertigen Präparate enthalten pro Dosiseinheit beispielsweise 150 bis 250 mg an Extraktstoffen bei einem Gesamtgewicht der Tablette oder des Drageekerns von 300 bis 500 mg.

Feste Arzneiformen, die unter Verwendung des erfindungsgemäßen Granulats hergestellt werden, zeigen auch bei längerer Lagerung bei Raumtemperatur keine Verfärbung und besitzen eine verbesserte Konstanz der Qualitätsparameter.

Die nachfolgenden Beispiele erläutern die Erfindung näher:

a) Herstellung des Granulats:

Beispiel 1:

3000g einer Mischung aus gleichen Gewichtsteilen Avicel PH 101 (mikrokristalline Cellulose) und Polyplasdone (quervernetztes Polyvinylpyrrolidon) werden im Materialbehälter eines Wirbelschichtgranulators mit einem Nennvolumen von 7 Litern vorgelegt und durch Einströmen von trockener Luft mit einer Strömungsgeschwindigkeit von 500 m³ pro Stunde und einer Temperatur von 80° C eine gleichmäßige Wirbelschicht gebildet.

20 Liter einer 20%igen wässerigen Lösung der Extraktstoffe werden mit 1 Liter einer 20 % wässerigen Lösung von Kollidon K 25 (Polymer des 1-Vinyl-2-pyrrolidons) vermischt, mittels einer Schlauchpumpe einer Zweistoffdüse zugeführt und in die Wirbelschicht eingesprüht. Die Sprühgeschwindigkeit wurde so geregelt, daß sich im Wirbelschichtgranulator eine Wirbelschichttemperatur von 28 bis 30° C und ein stationärer Wassergehalt des Wirbelschichtgutes von etwa 15 Gew. % einstellt, wobei sich die eingesprühte und verdampfende Wassermenge die Waage halten und keine Agglomeration zu beobachten ist.

Zur Aufrechterhaltung dieser Bedingungen beträgt die mittlere Sprühgeschwindigkeit 70 g Lösung pro Minute. Nachdem etwa 3/4 des gesamten Lösungsvolumens eingesprüht wurden, wurde die Temperatur der Zuluft auf 30° C gesenkt und der Rest des Lösungsvolumens bei gleichbleibender Sprühgeschwindigkeit eingesprüht. Der stationäre Wassergehalt in der Wirbelschicht steigt nun an und bei einem Wassergehalt von 38 bis 40 Gew. % setzt die Agglomeration des Pulvers ein. Die Granulation wird bei diesem Wassergehalt solange fortgesetzt, bis sich aus der gesamten Pulvermenge ein Granulat mit einem Durchmesser von 0.06 mm bis 0.8 mm gebildet hat. Nach Beendigung des Einsprühens wird die Temperatur der Zuluft wieder auf 60 - 80° C erhöht und damit die Trocknungsphase eingeleitet. Die Granulattemperatur beträgt während des Trocknens 30 bis 32° C. Die Trocknung ist beendet, wenn die Temperatur des Granulats ansteigt. Zur Entfernung des Restwassers wird die Temperatur des Granulats für 15 Minuten auf 43° C gehalten. Man erhält so 7350 g eines Granulats mit folgenden Eigenschaften:

| | |
|---|---|
| Gehalt an Extraktstoffen | 544.2 mg/g Granulat |
| Kollidon K 25 | 27.3 mg/g Granulat |
| Avicel PH 101 | 204.0 mg/g Granulat |
| Polyplasdone | 204.0 mg/g Granulat |
| Restwassergehalt | etwa 20 mg/g Granulat |
| | entsprechend etwa 2 % |
| Durchmesser | von 0.08 bis 0.8 mm |

Beispiel 2:

Versuch 1 wird wiederholt, wobei als Füll- und Trägerstoff 3000 g mikrokristalline Cellulose (Avicel PH 101) verwendet werden und als Bindemittel der Lösung der Extraktstoffe 1 Liter einer 20 %-igen wässerigen Lösung des 1-Vinyl-2-pyrrolidonpolymers Kollidon 90 zugesetzt wurde.

Man erhält so ein Granulat mit folgenden Eigenschaften:

| | |
|---|---|
| Gehalt an Extraktstoffen | 544.2 mg/g Granulat |
| Kollidon 90 | 21.3 mg/g Granulat |
| Avicel PH 101 | 408 mg/g Granulat |
| Restwassergehalt | etwa 20 mg/ g Granulat |
| | entsprechend etwa 2 % |
| Durchmesser | von 0.08 bis 0.8 mm |

Beispiel 3:

15.000 g Avicel PH 101 (mikrokristalline Cellulose) und 10.000 g AC-DI-Sol (quervernetzte Natrium-carboxymethylcellulose) werden im Materialbehälter eines Wirbelschichtgranulators mit einem Nennvolumen von 60 Litern vorgelegt. Durch Zuluft mit einer Strömungsgeschwindigkeit von 800 m³/Stunde und einer Temperatur von 80° C wird eine gleichmäßige Wirbelschicht aufrecht erhalten. 166.6 Liter einer 20%igen wässerigen Lösung der Extraktstoffe werden mit einer Lösung von 1660 g Kollidon K 25 in 16.6 Liter Wasser vermischt, mittels einer Schlauchpumpe (3 Düsen mit 1,2 mm Düsenöffnung) zugeführt und in die Wirbelschicht eingesprüht. Bei einer Sprühgeschwindigkeit von ca. 600 g Actovegin-Kollidon-Lösung/Minute stellt sich in der Wirbelschicht ein stationärer Wassergehalt von ca. 15 % und einer Temperatur von 30 bis 32° C ein.

Nachdem 140 Liter unter diesen Bedingungen eingesprüht sind, wird die Zulufttemperatur auf Raumtemperatur gesenkt, und der Wassergehalt so erhöht und die Agglomerierphase eingeleitet. Nach Beendigung des Einsprühens wird das gebildete Granulat im Wirbelschichtgranulator bei einer Zulufttemperatur von 80° C getrocknet.

Das getrocknete Granulat hat folgende Eigenschaften:

| Schüttdichte | $0.53 \text{ g/cm}^3$ |
|---|---|
| Stampfdichte | $0.63 \text{ g/cm}^3$ |
| Fließfähigkeit | $12.30 \text{ g/cm}^2 \text{ sec.}$ |
| Wassergehalt | 1.6 % |
| Korngröße | 60 - 500 um |

Beispiel 4:

22.000 g mikrokristalline Cellulose (Avicel PH 101) werden im Materialbehälter eines Wirbelschichtgranulators mit einem Nennvolumen von 60 Litern vorgelegt. Durch Zuluft mit einer Strömungsgeschwindigkeit von 800 m³/Stunde und einer Temperatur von 70° C wird eine gleichmäßige Wirbelschicht aufrecht erhalten. 166.6 Liter einer 20 %-igen wässerigen Lösung von Actovegin (Extraktstoffe aus enteiweißtem Kälberblut) werden mit einer Lösung von 1660 g Kollidon 90 (Polymer des 1-Vinyl-2-pyrrolidon) in 16.6 Liter Wasser vermischt, mit einer Schlauchpumpe (3 Düsen mit 1.2 mm Düsenöffnung) zugeführt und in die Wirbelschicht eingesprüht. Bei einer anfänglichen Sprühgeschwindigkeit von ca. 600 g Actovegin-Kollidon 90-Lösung/Minute stellt sich in der Wirbelschicht ein stationärer Wassergehalt von ca. 15 % und eine Temperatur von 30 - 32 °C ein.

Nach 2.5 Stunden wird bei gleicher Zulufttemperatur die Sprühgeschwindigkeit auf 900 g/Minute erhöht und die Agglomerierphase verstärkt. Nach Beendigung des Einsprühens wird das gebildete Granulat im Wirbelschichtgranulator bei einer Zulufttemperatur von 70 °C getrocknet.

Das getrocknete Granulat hat folgende Eigenschaften:

| Schüttdichte | $0.53 \text{ g/cm}^3$ |
|---|---|
| Stampfdichte | $0.63 \text{ g/cm}^3$ |
| Fließfähigkeit | $12.30 \text{ g/cm}^3 \text{ sec.}$ |
| Wassergehalt | 1.6 % |
| Korngröße | 60 - 500 µm |

b) Verwendung des Granulats zur Herstellung von festen Arzneiformen

Beispiel 5:

Drageekerne

7200 g eines nach Beispiel 1 erhaltenen Granulats werden mit 60 g Magnesiumstearat und 40g Talkum vermischt und in einem Rundläufer bei einem Preßdruck von 78.5 - 150 N/mm² zu Drageekernen mit einem Gewicht von 365 mg verpreßt. Man erhält so Drageekerne, die folgende Eigenschaften aufweisen:

| Kerneigenschaften: | | Zusammensetzung: | |
|---|---|---|---|
| Durchmesser | 10.0 mm | Extraktstoffe | 200 mg |

7

| Dicke | 5,5 mm | Polyplasdone | 75 mg |
|---|---|---|---|
| Bruchfestigkeit | 115 N | Avicel PH 101 | 75 mg |
| Abrieb (4 min) | 20.1 % | Kollidon K 25 | 10 mg |
| Zerfall | 8 - 10 min | Talkum | 2 mg |
| | | Mg-Stearat | 3 mg |

Diese Drageekerne haben einen konstanten Gehalt an Extraktstoffen und zeigen bei Lagerung bei Raumtemperatur innerhalb von 24 Monaten keine Verschlechterung in der Qualität oder Verfärbung.

Beispiel 6:

Tabletten:

7200 g eines nach Beispiel 1 erhaltenen Granulats werden mit 60 g Magnesiumstearat und 40 g Talkum vermischt und in einem Rundläufer bei einem Preßdruck von 98.1 bis 196.2 N/mm$^2$ zu Tabletten mit einem Gewicht von 730 mg verpreßt. Man erhält so Tabletten, die folgende Eigenschaften aufweisen:

**Tabletteneigenschaften:**  **Zusammensetzung:**

| Oblong-Tablette | | Extraktstoffe | 400 mg |
|---|---|---|---|
| Länge | 18 mm | Polyplasdone | 150 mg |
| Breite | 7 mm | Avicel PH 101 | 150 mg |
| Dicke | 6,7 mm | Kollidon K 25 | 20 mg |
| Bruchfestigkeit | 180 N | Talkum | 4 mg |
| Abrieb (4 min) | 0.1 % | Mg-Stearat | 6 mg |
| Zerfall | 10 min | | |

**Ansprüche**

1. Verfahren zur gleichzeitigen Gewinnung der Trockensubstanz aus wässerigen Lösungen von Extraktstoffen aus enteiweißtem Kälberblut und Herstellung eines zu festen Arzneiformen verpreßbaren Granulats, das mindestens 50 Gew. % Extraktstoffe bezogen auf das Gesamttrockengewicht des Granulats, ein Bindemittel und pharmazeutisch verträgliche Füll- und Trägerstoffe enthält, dadurch gekennzeichnet, daß man in einem kontinuierlichen Prozeß in einem Wirbelschichtgranulator auf eine vorgegebene Menge der Füll-und Trägerstoffe in fluidisiertem Zustand ein die gewünschte Extraktstoffmenge enthaltendes Volumen einer konzentrierten wässerigen Lösung, die auf 10 Gew. Teile der Extraktstoffe zusätzlich 0.2 bis 2 Gew. Teile des Bindemittels in gelöstem Zustand enthält, aufsprüht, wobei man während des Aufsprühens des gesamten Lösungsvolumens oder zumindest des größeren Teiles davon die Temperatur des einströmenden Fluidisiergases und die Sprühgeschwindigkeit so einstellt, daß bei einer Wirbelschichttemperatur von 25 bis 30° C und einem Wassergehalt des Wirbelschichtgutes von 15 bis 20 Gew. % die eingesprühte und verdampfende Wassermenge einander etwa entsprechen, keine Formgebung stattfindet und das Wirbelschichtgut pulverförmig bleibt, zum Agglomerieren und Granulieren des so erhaltenen Mischguts noch während oder nach Beendigung des Aufsprühens der Extraktstoffe den Wassergehalt in der Wirbelschicht auf 35 bis 45 Gew. % erhöht und anschließend das gebildete Granulat nach Erreichen der gewünschten Granulatgröße durch Erhöhen der Temperatur des einströmenden Fluidisiergases bei einer Wirbelschichttemperatur von 30 bis höchstens 45° C der Kurzzeittrocknung unterwirft.

2. Verfahren nach Anspruch 1, dadurch gekennzeichnet, daß man 25 bis 45 Gew. % an Füll- und Trägerstoffen, bezogen auf das Gesamttrockengewicht des Granulats, im Wirbelschichtgranulator vorlegt.

3. Verfahren nach den Ansprüchen 1 oder 2, dadurch gekennzeichnet, daß man als Füll- und Trägerstoff mikrokristalline Cellulose oder quervernetzte Natriumcarboxymethylcellulose verwendet.

4. Verfahren nach den Ansprüchen 1 oder 2, dadurch gekennzeichnet, daß man als Füll- und Trägerstoffe eine Mischung aus gleichen Gewichtsteilen an mikrokristalliner Cellulose und quervernetztem Polyvinylpyrrolidon verwendet.

5. Verfahren nach einem der Ansprüche 1 bis 4, dadurch gekennzeichnet, daß man als Fluidisiergas Luft verwendet.

6. Verfahren nach einem der Ansprüche 1 bis 5, dadurch gekennzeichnet, daß man eine Lösung aufsprüht, die 15 bis 25 Gew. % Extraktstoffe enthält.

7. Verfahren nach einem der Ansprüche 1 bis 6, dadurch gekennzeichnet, daß man eine Lösung aufsprüht, die auf 10 Gew. Teile der Extraktstoffe 0.5 bis 1 Gew. Teil des Bindemittels enthält.

8. Verfahren nach einem der Ansprüche 1 bis 7, dadurch gekennzeichnet, daß man als Bindemittel Polymere des 1-Vinyl-2-pyrrolidon verwendet.

9. Verfahren nach einem der Ansprüche 1 bis 8, dadurch gekennzeichnet, daß man nach dem Aufsprühen des größeren Teil des Lösungsvolumens den Wassergehalt in der Wirbelschicht zum Agglomerieren und Granulieren durch Absenken der Temperatur des einströmenden Fluidisiergases und/oder Vergrößerung der Sprühgeschwindigkeit erhöht.

10. Verfahren nach einem der Ansprüche 1 bis 8, dadurch gekennzeichnet, daß man nach Beendigung des Aufsprühens der Extraktstoffe den Wassergehalt in der Wirbelschicht zum Agglomerieren und Granulieren durch Einsprühen von reinem Wasser erhöht.

11. Verfahren nach den Ansprüchen 9 oder 10, dadurch gekennzeichnet, daß man den Wassergehalt in der Wirbelschicht auf 38 bis 40 Gew. % erhöht.

12. Verfahren nach einem der Ansprüche 1 bis 11, dadurch gekennzeichnet, daß man das Granulat bei einer Wirbelschichttemperatur von 30 bis 32° C trocknet und den Restwassergehalt durch kurzzeitige Erhöhung der Temperatur auf 40 bis 43° C entfernt.

13. Verfahren nach einem der Ansprüche 1 bis 12, dadurch gekennzeichnet, daß man ein Granulat mit einem Extraktstoffgehalt von 53 - 70 Gew. % bezogen auf das Gesamttrockengewicht des Granulats herstellt.

14. Verfahren nach einem der Ansprüche 1 bis 13, dadurch gekennzeichnet, daß man ein Granulat mit einem Durchmesser im Bereich von 0.05 bis 1 mm herstellt.

15. Zu festen Arzneiformen verpreßbares Granulat, welches mindestens 50 Gew% Extrakstoffe aus enteiweißtem Kälberblut, bezogen auf das Gesamttrockengewicht, ein Bindemittel und pharmazeutisch verträgliche Füll- und Trägerstoffe enthält, hergestellt nach dem Verfahren gemäß den Ansprüchen 1 bis 14.

16. Verwendung eines nach einem der Ansprüche 1 bis 14 erhaltenen Granulats zur Herstellung von festen pharmazeutischen Präparaten die Extraktstoffe aus enteiweißtem Kälberblut als Wirksubstanz enthalten.

17. Verwendung nach Anspruch 16, dadurch gekennzeichnet, daß man das Granulat gegebenenfalls mit weiteren in der Galenik üblichen Hilfsstoffen zu Tabletten, Dragees oder Drageekernen verpreßt.

18. Pharmazeutische Präparate, enthaltend ein Granulat gemäß Anspruch 15.

9

## Claims

1. Method for simultaneously obtaining the dry matter from aqueous solutions of extract substances from deproteinised calves blood and producing granules which can be compressed to give solid medicament forms and contain at least 50% by weight of extract substances, relative to the total dry weight of the granules, a binder and pharmaceutically acceptable fillers and carriers, characterised in that in a continuous process in a fluidised-bed granulator a volume, containing the desired quantity of extract substances, of a concentrated aqueous solution, which additionally contains 0.2 to 2 parts by weight of the binder in the dissolved state per 10 parts by weight of extract substances is sprayed onto a given quantity of the fillers and carriers in the fluidised state, the temperature of the fluidising gas flowing in and the spraying rate being adjusted, during the spraying-in of the total solution volume or at least during the major part thereof, in such a way that, at a fluidised-bed temperature of 25 to 30 °C and at a water content of 15 to 20% by weight in the fluidised-bed material, the quantity of water sprayed in and that being evaporated approximately correspond to each other, no shapes are formed and the fluidised-bed material remains pulverulent, in order to agglomerate and granulate the mixed material thus obtained the water content of the fluidised bed is increased to 35 to 45% by weight, while the extract substances are still being sprayed in or after completion thereof, and then, when the desired granule size has been reached, the granules formed are briefly dried at a fluidised-bed temperature of 30 up to at most 45 °C by increasing the temperature of the fluidising gas flowing in.

2. Method according to Claim 1, characterised in that 25 to 45% by weight of fillers and carriers, relative to the total dry weight of the granules, are placed into the fluidised-bed granulator.

3. Method according to Claim 1 or 2, characterised in that the fillers and carriers are microcrystalline cellulose or cross-linked sodium carboxymethylcellulose.

4. Method according to Claim 1 or 2, characterised in that the fillers and carriers are a mixture of equal parts by weight of microcrystalline cellulose and cross-linked polyvinylpyrrolidone.

5. Method according to Claims 1 to 4, characterised in that the fluidising gas is air.

6. Method according to any of Claims 1 to 5, characterised in that the solution sprayed on contains 15 to 25% by weight of extract substances.

7. Method according to any of Claims 1 to 6, characterised in that the solution sprayed on contains 0.5 to 1 part by weight of binder per 10 parts by weight of extract substances.

8. Method according to any of Claims 1 to 7, characterised in that the binder is a polymer of 1-vinyl-2-pyrrolidone.

9. Method according to any of Claims 1 to 8, characterised in that after the major part of the solution volume has been sprayed on, the water content of the fluidised bed is raised, for the agglomeration and granulation, by lowering the temperature of the fluidising gas flowing in and/or increasing the spraying rate.

10. Method according to any of Claims 1 to 8, characterised in that after spraying-on of the extract substances has been completed, the water content of the fluidised bed is raised, for the agglomeration and granulation, by spraying in pure water.

11. Method according to Claim 9 or 10, characterised in that the water content of the fluidised bed is increased to 38 to 40% by weight.

12. Method according to any of Claims 1 to 11, characterised in that the granules are dried at a fluidised-bed temperature of 30 to 32 °C and the residual water content is removed by briefly raising the temperature to 40 - 43 °C.

13. Method according to any of Claims 1 to 12, characterised in that the prepared granules contain 53 to 70% by weight of extract substances, relative to the total dry weight of the granules.

14. Method according to any of Claims 1 to 13, characterised in that the prepared granules have a diameter in the range from 0.05 to 1 mm.

15. Granules which can be compressed to give solid medicament forms and contain at least 50% by weight of extract substances from deproteinised calves blood, relative to the total dry weight, a binder and pharmaceutically acceptable fillers and carriers, produced by the method according to Claims 1 to 14.

16. Use of granules obtained according to any of Claims 1 to 14 for the production of solid pharmaceutical preparations which contain extract substances from deproteinised calves blood as active substance.

17. Use according to Claim 16, characterised in that the granules may be compressed with further auxiliaries customary in pharmacy to give tablets, coated tablets or tablet cores.

18. Pharmaceutical preparations containing the granules according to Claim 15.

**Revendications**

1. Procédé pour isoler simultanément une substance sèche à partir d'une solution aqueuse d'extraits de sang de veau déprotéiné et pour préparer un granulat comprimable en formes médicamenteuses solides, renfermant au moins 50% en masse d' extraits calculé sur la masse totale sèche du granulat, un liant et des substances adjuvantes et vectrices pharmaceutiquement acceptables, caractérisé en ce qu' on injecte dans un processus en continu conduit dans un granulateur à lit fluidisé sur une quantité prédéterminée des substances adjuvantes et vectrices à l'état fluidisé un volume renfermant la quantité d'extraits souhaitée d'une solution aqueuse concentrée, renfermant par 10% en masse d'extraits supplémentairement 0,2 à 2 parties en poids de liant dilué, en ce qu'on ajuste la température du gaz de fluidisation introduit et le débit d'injection pendant l'injection de l'ensemble de la solution ou tout au moins la plus grande partie de celle-ci de telle façon qu' il ne se produise aucune prise en masse et que la masse fluidisée reste poudreuse à une température du lit fluidisé de 25 à 30 °C et une proportion d'eau de la masse fluidisée de 15 à 20%, pour élever la quantité d'eau à un pourcentage de 35 à 45% en masse lors de l'agglomération et de la granulation du mélange ainsi obtenu, soit pendant, soit à la fin de l'injection des extraits, et ensuite entreprendre un court séchage de granulat formé après obtention de la granulation souhaitée par élévation de la température du gaz de fluidisation introduit à une température du lit fluidisé de 30 à 45 °C au plus.

2. Procédé selon la revendication 1, caractérisé en ce qu'on utilise 25 à 45% en masse de substance adjuvantes et vectrices, calculé sur la masse totale sèche du granulat, dans le granulateur à lit fluidisé.

3. Procédé selon l'une des revendications 1 ou 2, caractérisé en ce qu'on utilise comme substances adjuvantes et vectrices la cellulose microcristalline ou la carboxyméthylcellulose de sodium réticulée.

4. Procédé selon l 'une des revendications 1 ou 2, caractérisé en ce qu 'on utilise comme substances adjuvantes et vectrices une quantité égale de cellulose microcristalline et de polyvinylpyrrolidone réticulée.

5. Procédé selon l'une des revendications 1 à 4, caractérisé en ce qu'on utilise l'air comme gaz de fluidisation.

6. Procédé selon l'une des revendications 1 à 5, caractérisé en ce qu'on injecte une solution renfermant 15 à 25% d'extraits.

7. Procédé selon l'une des revendications 1 à 6, caractérisé en ce qu'on injecte une solution renfermant 0,5 à 1 partie en poids de liant par 10 parties en poids d'extraits.

8. Procédé selon l'une des revendications 1 à 7, caractérisé en ce qu'on utilise comme liant un polymère du 1-vinyl-2-pyrrolidone.

9. Procédé selon l'une des revendications 1 à 8, caractérisé en ce qu'on augmente la proportion d'eau

EP 0 223 033 B1

dans le lit fluidisé après injection de la plus grande partie de la solution pour agglomérer et granuler par diminution de la température du gaz de fluidisation introduit et/ou par augmentation de débit.

10. Procédé selon l'une des revendications 1 à 8, caractérisé en ce qu'on élève la proportion d'eau dans le lit fluidisé à la fin de l'injection des extraits pour agglomérer et granuler avec de l'eau pure.

11. Procédé selon les revendications 9 ou 10, caractérisé en ce qu'on élève la proportion d'eau dans le lit fluidisé de 38 à 40% en masse.

12. Procédé selon l'une des revendications 1 à 11, caractérisé en ce qu'on sèche le granulat à une température de lit fluidisé de 30 à 32 °C, et en ce qu'on élimine le reliquat d'eau par un court séchage en elevant la température à 40-43 °C.

13. Procédé selon l'une des revendications 1 à 12, caractérisé en ce qu'on prépare un granulat avec une quantité d'extrait de 53 à 70% en masse, calculée sur la masse totale sèche du granulat.

14. Procédé selon l'une des revendications 1 à 13, caractérisé en ce qu'on prépare un granulat ayant un diamètre de 0,05 à 1mm.

15. Granulat comprimable en formes médicamenteuses solides renfermant au moins 50% en masse d'extraits de sang de veau déprotéiné, calculé sur la masse totale sèche, un liant et des substances adjuvantes et vectrices pharmaceutiquement acceptables obtenu selon le procédé selon les revendications 1 à 14.

16. Utilisation d'un granulat obtenu selon le procédé des revendications 1 à 14 à la fabrication de préparations médicamenteuses solides, renfermant les extraits de sang de veau déprotéiné comme principe actif.

17. Utilisation selon la revendication, caractérisée en ce qu'on comprime le cas échéant le granulat avec des adjuvants habituels dans le processus galénique en comprimés, dragées ou noyaux de dragées.

18. Préparations pharmaceutiques renfermant un granulat selon la revendication 15.

12